# EUROPÄISCHE PATENTSCHRIFT

(11) **EP 4 429 608 B1**
(45) Veröffentlichungstag und Bekanntmachung des Hinweises auf die Patenterteilung: **08.04.2026**
(21) Anmeldenummer: 22817599.8
(22) Anmeldetag: 10.11.2022
(51) Int. Cl.: A61F 5/05, A61F 5/01, A61F 5/37

(54) **VERFAHREN ZUM BEFESTIGEN EINER ORTHESE UND ORTHESE**
METHOD FOR FASTENING AN ORTHESIS, AND ORTHESIS
PROCÉDÉ DE FIXATION D'UNE ORTHÈSE ET ORTHÈSE

(30) Priorität: 10.11.2021 DE 102021129271
(43) Veröffentlichungstag der Anmeldung: 18.09.2024
(73) Patentinhaber: Ottobock SE & Co. KGaA, 37115 Duderstadt (DE)
(72) Erfinder: VOLLBRECHT, Matthias, 37115 Duderstadt (DE); HOFFSÜMMER, Laura, 37115 Duderstadt (DE)
(74) Vertreter: Gramm, Lins & Partner Patent- und Rechtsanwälte PartGmbB
(86) Internationale Anmeldenummer: PCT/EP2022/081529
(87) Internationale Veröffentlichungsnummer: WO 2023/083986

(56) Entgegenhaltungen:
- EP-A2- 2 356 957
- DE-A1- 102011 119 397
- DE-U1- 20 114 446
- US-A- 4 862 878
- US-B2- 7 320 669

## Beschreibung

Die Erfindung betrifft ein Verfahren zum Befestigen einer Schulterorthese an einen Oberarm, wobei die Orthese eine formstabile Schale mit einer ventralen Lasche und einer dorsalen Lasche aufweist, wobei die ventrale Lasche und die dorsale Lasche mittels eines Spannelementes verbunden oder verbindbar sind. Die Erfindung betrifft zudem eine Schulterorthese zum Durchführen eines derartigen Verfahrens.

Orthesen sind orthopädietechnische Vorrichtungen, durch die ein Körperteil eines Trägers der Orthese geschützt, unterstützt und/oder gestärkt werden soll. Die vorliegende Erfindung betrifft Orthesen für die obere Extremität, die im proximalen Bereich des Oberarms und an der Schulter des Trägers angeordnet werden. Sie kommen beispielsweise bei Schlaganfallpatienten oder im Falle einer Plexus Parese zur Anwendung. Derartige Orthesen sind insbesondere dann von Vorteil, wenn die Oberarmmuskulatur des Trägers nicht ausreichend stark ist, um den Arm des Trägers am Schultergelenk zu halten. Die Orthesen sollen die Subluxation im Schultergelenk verringern und die Kraft, insbesondere das Gewicht des jeweiligen Armes aufnehmen und auf die Schulter und die gegenüberliegende, kontralaterale Seite übertragen. Derartige Orthesen können als Schulterorthese bezeichnet werden und werden beispielsweise auch eingesetzt, um die Schulter nach einer Verletzung oder an einer Schulter durchgeführten Operation zu stabilisieren und dennoch zumindest eine eingeschränkte Bewegung der Schulter zu ermöglichen und gegebenenfalls zu unterstützen. Dabei wird versucht, die geschwächte Schulter in einer optimalen Position zu schützen und gleichzeitig eine zumindest eingeschränkte Bewegung zu erlauben oder zu erleichtern.

Dazu weisen herkömmliche Schulterorthesen einen Grundkörper aus einem elastischen Material auf. Dieser verfügt beispielsweise über einen Schulteranteil, der im angelegten Zustand an der Schulter des Trägers der Schulterorthese anliegt. An diesem Schulteranteil befindet sich ein herkömmlicherweise tubusförmiger Armanteil, der um den Oberarm des Trägers der Schulterorthese herum angeordnet wird. Da der Grundkörper aus einem elastischen Material besteht, kann die Schulterorthese, ähnlich einem Hemdsärmel, einfach über den Arm gezogen werden, bis sie an der Schulter anliegt. Durch den wenigstens einen vorgesehenen Gurt, der um den Oberkörper des Trägers der Schulterorthese herum gelegt wird, wird der Grundkörper der Schulterorthese in dieser Position gehalten und gleichzeitig in vielen Ausgestaltungen eine Zugkraft auf zumindest den Schulterbereich des Grundkörpers ausgeübt. Dabei ist es möglich, den wenigstens einen Gurt an zumindest einer Stelle lösbar mit dem Grundkörper zu verbinden, um ein einfacheres Anlegen der Orthese zu erlauben.

Die US 4,862,878 A beschreibt eine Schulterorthese, die eine unterstützende Kraft auf die Schulter aufbringen soll, insbesondere wenn der entsprechende Arm angehoben wird. Dazu weist die Orthese ein über Rollen geführtes elastisches Band auf. Die EP 2 356 957 A2 beschreibt hingegen eine Orthese, durch die die Schulter ruhiggestellt und gerade nicht mehr bewegt werden soll.

Die US 7,320,669 B2 offenbart eine Schulterorthese, die ein pneumatisches Kissen aufweist, das gefüllt oder geleert werden kann, um einen Druck einzustellen, der auf eine Schulter ausgeübt werden soll. Die DE 201 14 446 U1 hingegen befasst sich mit einer Schulterorthese, die eine Außenrotation auf den Oberarm ausübt.

Wird eine Schulterorthese aus dem Stand der Technik angelegt, wird zunächst der Arm des Trägers der Schulterorthese in den Armanteil des Grundkörpers eingeführt und der Grundkörper entlang des Armes nach oben gezogen, bis er an der Schulter seine endgültige Position erreicht. Durch den einen Gurt, der beispielsweise mit einem seiner Enden unlösbar mit dem Grundkörper der Schulterorthese verbunden ist, kann nun eine Zugkraft auf den Grundkörper aufgebracht werden, die durch den Grundkörper auf die Schulter ausgeübt wird, an der er anliegt. An dem freien Ende des wenigstens einen Gurtes befindet sich vorteilhafterweise ein Befestigungselement, das beispielsweise ein Teil eines Klettverschlusses sein kann.

Das entsprechende Gegenstück befindet sich am Grundkörper der Schulterorthese, insbesondere am Schulteranteil dieses Grundkörpers. Dadurch ist es möglich, die effektive Länge des Gurtes und damit auch die von dem Gurt ausgeübte Zugkraft individuell auf den Träger der Schulterorthese einzustellen. Der Träger der Schulterorthese oder eine ihm helfende Person übt folglich einen Zug auf den wenigstens einen Gurt aus, bevor er das freie Ende des Gurtes mit dem Grundkörper der Schulterorthese verbindet. Auf diese Weise wird eine Zugkraft auf den Grundkörper der Schulterorthese und hier insbesondere auf den Schulteranteil der Schulterorthese ausgeübt. Da dieser jedoch aus einem elastischen Material besteht, gibt er diesen Zugkräften zumindest über einen bestimmten Bereich hinweg nach.

Der Grundkörper der Schulterorthese wird herkömmlicherweise direkt auf der Haut des Trägers der Schulterorthese angeordnet. Dabei kann die Innenseite des Grundkörpers beschichtet sein, um eine möglichst gute Haftung des Grundkörpers der Schulterorthese an der Haut des Trägers zu gewährleisten. Auf diese Weise wird ein Verrutschen der Schulterorthese während der Bewegung der Schulter verhindert oder zumindest erschwert. Nachteilig ist, dass dann, wenn der Gurt mit dem Grundkörper der Schulterorthese verbunden wird, eine Zugkraft auf diesen Grundkörper ausgeübt wird, unter der er sich verformt. Durch die erhöhte Haftreibung zwischen der Haut des Trägers der Schulterorthese und dem Grundkörper wird diese Verformung an die Haut des Patienten und damit an das eigentlich zu stützende Schulterelement weitergeleitet. Beim Schließen einer derartigen Schulterorthese kommt es folglich zu einer Bewegung der Schulter, die dadurch im Regelfall nach vorn gezogen wird. In dieser Haltung wird die Schulter dann durch die Schulterorthese gestützt und fixiert. Diese Haltung ist jedoch nicht die gewünschte optimale gesunde Haltung, sondern eine gegenüber dieser Haltung verschobene Haltung. Daher ist es nachteilig, die Schulter in dieser Position zu unterstützen und zu fixieren.

Das gleiche Problem stellt sich auch, wenn der Gurt nicht an einem Ende lösbar mit dem Grundkörper der Schulterorthese verbunden ist, sondern an beiden Enden unlösbar mit dem Grundkörper beispielsweise durch Annähen verbunden ist. Der Gurt kann auch einstückig mit dem Grundkörper ausgebildet sein, so dass er ebenfalls aus einem elastischen Material besteht. Beim Anlegen einer derartigen Schulterorthese kommt es dennoch zu einer Verformung und zu einem Verziehen des Grundkörpers, wodurch durch die große Haftreibung zwischen dem Grundkörper und der Haut des Trägers der Schulterorthese es wieder zu einer Bewegung und Verschiebung des Schultergelenks kommt, sodass auch in diesem Fall nicht gewährleistet ist, dass die Schulter in einer optimalen Position unterstützt wird.

Aus der DE 10 2011 119 397 A1 ist daher eine Schulterorthese bekannt, bei der Gurte, die die Kraft auf den Grundkörper der Orthese übertragen, aus einem und elastischen Material hergestellt sind. Dadurch wird zwar vermieden, dass es zu einer Verformung der Gurte und auch des Grundkörpers zumindest in dem Bereich kommt, der mit den Gurten verbunden ist, dennoch wird die Kraft, die beim Spannen der Orthese aufgebracht wird, zu einer Verformung und Verschiebung des Oberarms relativ zur Schulter des Trägers führen.

Der Erfindung liegt daher die Aufgabe zugrunde, die Nachteile aus dem Stand der Technik zu beheben oder zumindest zu verringern.

Die Erfindung löst die gestellte Aufgabe durch ein Verfahren zum Befestigen einer Orthese der oben genannten Art, dass die folgenden Schritte aufweist:
- Anlegen der formstabilen Schale an einen Oberarm,
- Spannen des Spannelementes, sodass eine Zugkraft auf die ventrale Lasche und die dorsale Lasche ausgeübt wird, die Richtung proximal wirkt.

Die formstabile Schale der Orthese ist vorzugsweise so ausgebildet, dass ihre geometrische Form an den Oberarm angepasst ist. Dies kann durch eine individuell an den Oberarm des jeweiligen Trägers angepasste Schale oder durch eine generische Schale erreicht werden, die allgemein an einen Oberarm angepasst ist. Vorzugsweise wird die formstabile Schale nicht direkt auf der Haut des Trägers positioniert, was aufgrund der Formstabilität insbesondere bei Bewegungen des Oberarms zu Schmerzen oder zumindest einem unangenehmen Druckgefühl führen kann. Dies ist besonders wahrscheinlich, wenn die formstabile Schale nicht individuell an den Oberarm angepasst ist.

Vorzugsweise verfügt die formstabile Schale an einer dem Oberarm zugewandten Seite über ein Polsterelement. Das Polsterelement ist Teil der Schale und so an der formstabilen Schale angeordnet, dass es mit dem Oberarm in Kontakt kommt, wenn die Schale an dem Oberarm angelegt ist. Das Polsterelement ist vorzugsweise flexibel, besonders vorzugsweise elastisch ausgebildet. In einer Ausgestaltung der Erfindung ist das Polsterelement als textiles Polsterelement ausgebildet.

Ein solches Polsterelement, insbesondere ein textiles Polsterelement, selbst ist folglich nicht zwangsläufig formstabil ausgebildet. Die Formstabilität der formstabilen Schale wird in diesem Fall nicht durch das Polsterelement, sondern ein anderes, formstabiles Bauteil der Schale hervorgerufen. Das Polsterelement, insbesondere ein textiles Polsterelement, kann deutlich größer als das formstabile Bauteil der formstabilen Schale ausgebildet sein. Das bedeutet, dass das Polsterelement mit einer deutlich größeren Fläche des Arms in Kontakt kommt, als es das formstabile Bauteil der formstabilen Schale tun würde.

Vorzugsweise ist die formstabile Schale derart geformt, dass sie den Oberarm zumindest teilweise umgibt. Vorzugsweise ist sie über einen Gurt oder ein ähnliches Element verschließbar, sodass die den Oberarm vollständig umgibt. Der Gurt oder das ähnliche Element ist in diesem Fall Teil der formstabilen Schale. Ein solcher Gurt kann beispielsweise mit einem Ende an der formstabilen Schale befestigt, vorzugsweise unlösbar befestigt, besonders bevorzugt angenäht sein und am gegenüberliegenden Ende ein Formschlusselement aufweisen, das eingerichtet ist, mit einem korrespondierend ausgebildeten zweiten Formschlusselement zusammenzuwirken, das beispielsweise an der formstabilen Schale befestigt ist. Nach dem Schließen eines solchen Gurtes wird der Oberarm des Trägers von der formstabilen S vollständig umgeben. Vorzugsweise kommt es dabei zumindest auch zu einer reibschlüssigen Verbindung zwischen der formstabilen Schale, vorzugsweise dem Polsterelement und dem Oberarm, sodass eine auf die Schale wirkende Kraft auf den Oberarm übertragen wird. Das Polsterelement der formstabilen Schale ist vorzugsweise aus einem elastischen Material hergestellt, das vorzugsweise beim Anlegen der Orthese an den Oberarm gespannt wird. Dadurch wird die Reibschlüssigkeit der Verbindung zwischen der Schale und dem Oberarm verstärkt. Die formstabile Schale, vorzugsweise das Polsterelement, ist vorzugsweise auf der dem Oberarm zugewandten Seite mit einer rutschhemmenden Beschichtung, beispielsweise einer Silikon-Beschichtung versehen.

Nach dem Anlegen der formstabilen Schale an den Oberarm erstrecken sich die dorsale Lasche und die ventrale Lasche jeweils nach proximal, bei einem nach unten hängenden Oberarm also nach oben. Oberhalb der Schulter sind sie miteinander verbunden oder verbindbar. Dabei ist es nicht notwendig, dass in diesem Zustand bereits eine in Richtung proximal wirkende Kraft auf den Oberarm übertragen wird. Dies geschieht vorzugsweise erst durch das Spannen des Spannelementes. Durch die besondere Ausgestaltung dieses Spannelementes wird jedoch anders als bei Orthesen aus dem Stand der Technik keine einseitige Kraft auf das am Oberarm anliegende Element der Orthese ausgeübt. Vielmehr wird die Zugkraft sowohl auf die ventrale Lasche als auch auf die dorsale Lasche ausgeübt. Dadurch kommt es zu einer gleichmäßigen Kraftübertragung und somit zu einer lediglich nach proximal wirkenden Kraft.

Aus dem Stand der Technik sind beispielsweise Gurte bekannt, die mit einem Ende an einer der beiden Laschen, beispielsweise an der dorsalen Lasche, unlösbar befestigt, beispielsweise angenäht sind. Am gegenüberliegenden Ende befindet sich ein Klettverschlusselement, das mit einem korrespondierenden Klettverschlusselement zusammenwirken kann, welches sich vorzugsweise an der anderen Lasche, beispielsweise an der ventralen Lasche, befindet. Zum Spannen eines solchen Spannelementes wird eine Zugkraft auf den Gurt ausgeübt, die ausschließlich auf die dorsale Lasche wirkt. Erst nachdem die beiden Klettverschlusselemente zusammenwirken und es zu einer formschlüssigen Verbindung kommt, wird auch eine Kraft auf die jeweils andere Lasche, in diesem Fall also die ventrale Lasche, ausgeübt. Beim Spannen erfolgt also keine gleichzeitige Krafteinleitung in die beiden Laschen. Dies ist bei der vorliegenden Erfindung anders. Hier kommt es bereits beim Spannen des Spannelementes zu einer Einleitung der Zugkraft sowohl in die ventrale Lasche als auch in die dorsale Lasche.

Vorzugsweise werden die ventrale Lasche und die dorsale Lasche vor dem Spannen des Spannelementes miteinander verbunden, besonders bevorzugt aneinander befestigt. Dies kann beispielsweise durch Formschlusselemente geschehen, die an den beiden Laschen angeordnet sind und miteinander wechselwirken können. In einer besonders einfachen Ausgestaltung ist ein Klettverschlusselement an einer der beiden Laschen und das entsprechende Klettverschluss-Gegenelement an der jeweils anderen Lasche angeordnet. Alternativ dazu kann auch ein Verbindungselement, beispielsweise ein Stoffstreifen oder ein Textilstreifen, ein Band oder ein Gurt verwendet werden, der mit den beiden Laschen verbindbar oder verbunden ist, um die beiden Laschen miteinander zu verbinden. Wichtig ist, dass dies vollständig oder nahezu vollständig ohne eine wirkende Zugkraft auf die Laschen geschehen kann und somit lediglich dazu dienen kann, die beiden Laschen in der gewünschten Position zu halten. Die nach proximal wirkende Kraft, die für einen guten Sitz und für eine Funktionsfähigkeit der Orthese wichtig ist, wird vorzugsweise noch nicht beim Verbinden der beiden Laschen erzeugt, sondern wird erst durch das Spannen des Spannelementes hervorgerufen.

Besonders bevorzugt werden die ventrale Lasche und die dorsale Lasche oberhalb der Schulter einander verbunden, vorzugsweise aneinander befestigt. Notwendig ist dies jedoch nicht. Eine Befestigung oder ein Verbinden kann auch in jeder anderen Stelle erfolgen.

Die Erfindung löst die gestellte Aufgabe zudem durch eine Orthese zum Durchführen eines Verfahrens der oben beschriebenen Art, wobei diese Orthese eine formstabile Schale mit einer ventralen Lasche und einer dorsalen Lasche aufweist, wobei sich die Orthese dadurch auszeichnet, dass sie ein Spannelement aufweist, durch das eine Zugkraft auf die ventrale Lasche und die dorsale Lasche aufbringbar ist, wobei diese Zugkraft vergrößerter bei ist, indem das Spannelement gespannt wird. Wie bereits dargelegt ist beim Vergrößern der Zugkraft durch Spannen des Spannelementes wichtig, dass die Zugkraft auf beide Laschen gleichzeitig wirkt und entsprechend vergrößert wird.

Eine formstabile Schale ist beispielsweise eine Schale, die ohne die Einwirkung äußerer Kräfte mit Ausnahme der Schwerkraft ihre Form behält. Wird eine formstabile Schale beispielsweise auf einer Fläche abgelegt, behält sie zumindest weitestgehend, vorzugsweise jedoch vollständig ihre Form. Eine aus einem einfachen Textil bestehende Schale hingegen würde in sich zusammenfallen. Die formstabile Schale weist zumindest abschnittsweise einen rohrförmigen oder teilrohrförmigen Bereich auf. Ein rohrförmiger Bereich umfasst den Oberarm des Trägers vollständig, also über den gesamten Umfang. Ein teilrohrförmiger Abschnitt umfasst den Oberarm nur über einen Teil des Umfangs. Der rohrförmige oder teilrohrförmige Bereich der Schale ist so ausgebildet, dass er auch im abgelegten zustand seine geöffnete Form beibehält. Die formstabile Schale und insbesondere der rohrförmige oder teilrohrförmige Bereich ist vorzugsweise so ausgeführt, dass der Benutzer diese mit dem gesunden Arm möglichst einfach auf den mit der Orthese zu versorgenden Arm einfädeln kann bis sie die gewünschte Endposition am Oberarm erreicht. In besonderer Ausführung ist die Schale so formstabil ausgeführt, dass die Schale ein Schwingen des Oberarms, an dem sie angelegt ist, vermindert und/oder dämpft. Besonders bevorzugt unterdrückt sie ein Überschwingen. Vorzugsweise wird das Schwingen um 25% - 50% vermindert. Dadurch wird die Armbewegung an eine natürliche Bewegung, insbesondere beim Gehen an das natürliche Gangbild (Schwingen der Arme) angepasst.

Die geometrische Form der Schale ist dabei vorzugsweise an den Oberarm angepasst, an den die formstabile Schale angelegt werden soll. Die formstabile Schale ist insbesondere derart ausgeführt, dass sie Scherkräfte aufnehmen kann, die quer zur Dicke des Materials eingeleitet werden. Diese Scherkräfte treten beispielsweise auf, wenn die Orthese beim Anlegen an den Oberarm gegen die Reibung der Haut in die Endposition bewegt wird. Dabei verformt sich die formstabile Schale vorzugsweise nicht. Beim Anlegen der Orthese an den Oberarm kommt es insbesondere dann zur Reibung mit der Haut des Arms des Trägers, wenn die Orthese einen den Oberarm vollständig umfassenden Bereich aufweist und die Orthese auf den Arm aufgefädelt wird. Dann kommt es oft bereits im Bereich des Unterarms und danach im Bereich des Oberarms zu engem Kontakt zwischen der Orthese und der Haut des Trägers. Eine rein textile Orthese würde sich dadurch in Falten legen und könnte nicht ohne weiteres in die Endposition bewegt werden. Dies wäre nur möglich, indem eine Zugkraft auf das obere Ende des Textils aufgebracht wird.

Durch die formstabile Eigenschaft, Scherkräfte aufnehmen zu können, kann die Orthese auf den Arm aufgeschoben werden, wenn beispielsweise der rohrförmige oder teilrohrförmige Bereich der Schale bewegt wird.

Die formstabile Schale kann beispielsweise aus einem Kunststoff, einem faserverstärkten Kunststoff, beispielsweise GFK oder CFK, oder einem Metall, beispielsweise Aluminium, hergestellt sein. Vorzugsweise verfügt die formstabile Schale über ein formstabiles Schaumelement. Das Schaumelement kann an der der Haut des Trägers zugewandten Seite mit dem Polsterelement verbunden sein. In einer bevorzugten Ausgestaltung ist das Schaumelement auf der der Haut des Trägers zugewandten Seite mit einem Textil verbunden. Das Textil soll für ein angenehmes Tragegefühl sorgen. Auf der entgegengesetzten Seite des Schaumelementes kann eine weitere Schicht aus einem Kunststoff oder einem textil aufgebracht sein, um die Anfälligkeit für äußere Einflüsse, wie beispielsweise Verschmutzungen zu reduzieren. Das Schaumelement hat vorzugsweise ein Biegemodul von wenigstens 7 MPa, vorzugsweise von wenigsten 10 MPa, besonders bevorzugt von wenigsten 13 MPa und von höchstens 100 MPa auf.

Die dorsale Lasche verläuft dorsal vorzugsweise bis kurz unter die Schulterblattgräte (Spina scapulae). Der Abstand zur Schulterblattgräte beträgt vorzugsweise weniger als 5 cm, besonders bevorzugt weniger als 2 cm. Der dorsale Vorsprung bildet das Gegenlager zu der nach ventral auslaufenden ventralen Lasche am Oberarm. Dieser Verlauf der Laschen grenzt eine mögliche Pendelbewegung des Oberarmes nach hinten ein und positioniert den Oberarm. Die ventrale Lasche der Schale erstreckt sich vorzugsweise am Schultergürtel bis kurz vor das Schlüsselbein. Der Abstand zum Schlüsselbein beträgt vorzugsweise weniger als 5 cm, besonders bevorzugt weniger als 2 cm. Die ventrale Lasche dient insgesamt zum Anheben des Armes wenn das Spannelement gespannt wird. Die Spange kann individuell an die anatomischen Gegebenheiten des Anwenders angepasst werden. Dazu wird beispielsweise eine vorkonfektionierte Schale eingearbeitet und nachbearbeitet oder eine Schale beispielsweise mittels additiver Fertigung oder handwerklicher Herstellung individuell gefertigt.

Die Orthese verfügt vorzugsweise über wenigstens einen, bevorzugt zwei Haltegurte, die vorzugsweise etwa in der Mitte des Oberarms des Trägers angeordnet sind und sich zu einem den Oberkörper umlaufenden Oberkörpergurt erstrecken. Diese dienen der Reduzierung/Eingrenzung des Pendelns des Armes nach vorne oder hinten. Diese Gurte können in der Länge individuell eingestellt werden.

Vorzugsweise weist das Spannelement einen Gurt auf, der mit einem Ende an einer ersten Lasche befestigt ist, mit dem zweiten Ende an der ersten Lasche befestigbar ist und durch eine Umlenkschlaufe an der zweiten der beiden Laschen geführt wird. Wird ein solches Spannelement gespannt, wird das zweite Ende, das noch nicht an einer ersten Lasche befestigt ist, durch die Umlenkschlaufe geführt. Anschließend kann eine Zugkraft auf dieses zweite Ende des Spannelementes ausgeübt werden. Diese Zugkraft führt zu einer Zugkraft auf beide Laschen, da sie einerseits über die Umlenkschlaufe auf die zweite Lasche und über das an der ersten Lasche befestigte erste Ende des Gurtes auf die erste Lasche übertragen wird. Ist eine ausreichende Zugkraft aufgebracht, kann das zweite Ende des Gurtes an der ersten Lasche befestigt werden.

Alternativ oder zusätzlich dazu verfügt das Spannelement über ein Zugseil, dass mit der ventralen Lasche und der dorsalen Lasche verbunden ist und dessen wirksame Länge veränderbar ist. Ein solches Zugseil kann auch in Form eines Drahtes vorliegen. Vorzugsweise verfügt ein solches Spannelement über ein Betätigungselement, durch das die wirksame Länge des Zugseils verändert werden kann. Ein solches Element wird beispielsweise von der Firma BOA angeboten. Das Betätigungselement ist in diesem Fall ein Drehelement, beispielsweise ein Drehknopf, der um eine Drehachse gedreht wird. Dabei wird beispielsweise das Zugseil auf eine Spindel oder Spule aufgewickelt und so dessen wirksame Länge, also insbesondere die Abstände zwischen dem Betätigungselement und dessen Spindel oder Spule einerseits und den Positionen, an denen das Zugseil mit der jeweiligen Lasche verbunden ist, reduziert. Dadurch wird eine Zugkraft auf die Lasche und damit auf die formstabile Schale der Orthese ausgeübt.

In einer bevorzugten Ausgestaltung wird das Zugseil durch eine Schlaufe an der ventralen Lasche und/oder eine Schlaufe an der dorsalen Lasche geführt. Auf diese Weise ist eine besonders einfache Befestigung und Verbindung zwischen der jeweiligen Lasche und dem Zugseil erreichbar, die konstruktiv besonders einfach hergestellt werden kann. In einer bevorzugten Ausgestaltung wird das Zugseil über wenigstens eine Umlenkrolle, besonders bevorzugt über mehrere Umlenkrollen geführt. Dadurch wird der Effekt eines Flaschenzuges erreicht, so dass die benötigte Kraft auch für motorisch eingeschränkte oder körperlich schwache Träger der Orthese aufgebracht werden kann.

In einer bevorzugten Ausführung ist an der formstabilen Schale ein Versteifungselement angeordnet. Damit wird die formstabile Schale verstärkt. Das Versteifungselement ist beispielsweise als zweite Schale oder als wenigstens eine Strebe ausgebildet. Das Versteifungselement wird mit der ersten formstabilen Schale vorzugsweise unlösbar verbunden, beispielsweise angeklebt oder angeschweißt. Alternativ wird das Versteifungselement lösbar mit der formstabilen Schale verbunden, beispielsweise durch Stecken oder die Verwendung einer Klettverbindung. Vorzugsweise lassen sich auf diese Weise die formstabile Schale und das Versteifungselement individuell an den Patienten anpassen. In einer bevorzugten Ausführung ist die formstabile Schale weniger formstabil als das Versteifungselement.

Das Versteifungselement kann auch als Spange bezeichnet werden und wird vorzugsweise fest mit der formstabilen Schale verbunden. Das Versteifungselement verfügt vorzugsweise über einen dorsalen Vorsprung und/oder einen ventralen Vorsprung. Der dorsale Vorsprung verläuft dabei entlang der dorsalen Lasche und der ventrale Vorsprung entlang der ventralen Lasche. Vorzugsweise ist das Versteifungselement als Schale ausgebildet. Das Versteifungselement ist vorzugsweise an der formstabilen Schale angeordnet. Dies geschieht vorzugsweise an der dem Oberarm abgewandten Seite. Vorzugsweise ist das Versteifungselement aus einem Metall, beispielsweise Aluminium oder Stahl, oder aus einem Kunststoff, einem faserverstärkten Kunstsoff, beispielsweise GFK oder CFK, hergestellt sein.

Vorzugsweise ist das Spannelement direkt oder indirekt, beispielsweise über einen Gurt, an dem Versteifungselement befestigt. Bevorzugt ist das Spannelement an den beiden Vorsprüngen des Versteifungselementes direkt oder indirekt, beispielsweise über einen Gurt, befestigt.

Vorzugsweise ist an der formstabilen Schale und/oder dem Versteifungselement wenigstens ein Element eines orthopädietechnischen Schienensystems befestigt oder befestigbar. Dies ist insbesondere dann von Vorteil, wenn beispielsweise auch ein Unterarm durch die Orthese gestützt, unterstützt oder geschützt werden soll. Das Element des Schienensystems erstreckt sich dann in die gewünschte Richtung und kann beispielsweise einen Unterarm in einer bestimmten und gewünschten Position fixieren oder unterstützen. Bevorzugt ist das Element des Schienensystems in unterschiedlichen Positionen und/oder unterschiedlichen Orientierungen an der formstabilen Schale der Orthese befestigbar. Dies kann durch einen Klemmmechanismus, durch einen Clips- oder Schnapp-Mechanismus oder durch Schraubverbindungen geschehen.

Besonders bevorzugt ist das Schienensystem Teil der Orthese und weist wenigstens ein Körperanlageelement, vorzugsweise ein Unterarmelement zum Anlegen an einen Unterarm und/oder ein Handelement zum Anlegen an eine Hand auf. Besonders bevorzugt verfügt das Schienensystem über wenigstens zwei Elemente, die mittels eines Gelenkes miteinander verbunden positioniert sind. Vorteilhafterweise verfügt das Schienensystem über wenigstens einen Aktuator, der eingerichtet ist, ein Element des Schienensystems relativ zu einem anderen Element des Schienensystems zu bewegen.

Durch die Anbindung eines Schienensystems an der formstabilen Schale und/oder dem Versteifungselement werden Kräfte von einem Ellenbogen, eines Unterarmes und/oder einer Hand zumindest teilweise, vorzugsweise jedoch vollständig auf das Spannelement übertragen. Dadurch wirkt die durch diese Kräfte, die beispielsweise durch das Gewicht des Arms und der Orthese hervorgerufen werden können, hervorgerufene Belastung nicht oder nicht nur auf den Oberarm oder das Schultergelenk, sondern wird zumindest teilweise, vorzugsweise vollständig, über das Spannelement abgeleitet. Diese Belastung wirkt in dem abgeleiteten Umfang also nicht als Zugkraft auf den Oberarm, sondern als Druckkraft, die von oben auf die Schulter wirkt.

Das Gelenk des orthopädietechnischen Schienensystems ist vorzugsweise ein Ellenbogengelenk, das besonders bevorzugt derart angeordnet ist, dass seine Schwenkachse mit der Schwenkachse des natürlichen Ellenbogens des Trägers der Orthese übereinstimmt. Anders als bei einer textilen Orthese ist eine Verschiebung oder Verdrehung des Gelenks relativ zu dem Körperteil des Trägers der Orthese nicht oder nur sehr wenig möglich. Dadurch kann eine einmal eingerichtete Position und Orientierung des Gelenkes lange aufrecht erhalten werden, wodurch die Funktionsfähigkeit der Orthese erhalten bleibt.

Dies gilt nicht nur für Ellenbogengelenke, sondern prinzipiell für alle Elemente des orthopädietechnischen Schienensystems, die direkt oder indirekt an der formstabilen Schale und/oder dem Versteifungselement angeordnet sind. Es gilt also beispielsweise für Fixierungsschienen, Ellenbogen- und Handgelenken. Sie alle sind relativ zu dem Körperteil des Trägers der Orthese ausrichtbar und durch die formstabile Ausgestaltung der einzelnen Komponenten in dieser Ausrichtung stabil haltbar.

Vorzugsweise ist an einer dem Oberarm zugewandten Seite der formstabilen Schale ein flexibles, vorzugsweise elastisches, Polsterelement angeordnet, das vorzugsweise ein textiles Polsterelement ist.

Bevorzugt sind die dorsale Lasche und/oder die ventrale Lasche Teil des Polsterelementes oder sie sind an diesem befestigt, vorzugsweise angenäht oder angeklebt.

Bevorzugt verbindet die formstabile Schale die ventrale Lasche und die dorsale Lasche auf der medialen Seite des Armes. Dies bedeutet, dass sich die Schale zwischen dem Arm und dem Oberkörper des Trägers erstreckt, wenn der Arm entspannt herunterhängt. Die entgegengesetzte Seite, also die laterale Seite, ist bevorzugt offen ausgebildet. Die dort befindliche Öffnung kann durch einen oder mehrere Gurte überspannt oder überbrückt werden, besonders bevorzugt ist jedoch kein Gurt vorhanden.

Vorzugsweise ist das Gelenk, beispielsweise das Ellenbogengelenk medial am Arm angeordnet. Dies ist besonders einfach möglich, wenn sich die formstabile Schale medial befindet.

Mithilfe der beiliegenden Figuren werden nachfolgend einige Ausführungsbeispiele der vorliegenden Erfindung näher erläutert. Es zeigt
Figur 1 und 2 - schematische Darstellungen einer Orthese gemäß einem ersten Ausführungsbeispiel der vorliegenden Erfindung
Figur 3 - eine schematische Darstellung einer Orthese gemäß einem weiteren Ausführungsbeispiel,
Figuren 4 bis 6 - eine Orthese gemäß einem weiteren Ausführungsbeispiel der vorliegenden Erfindung in unterschiedlichen Ansichten und
Figuren 7 und 8 - eine Orthese gemäß einem weiteren Ausführungsbeispiel der vorliegenden Erfindung im angelegten und nicht angelegten Zustand.

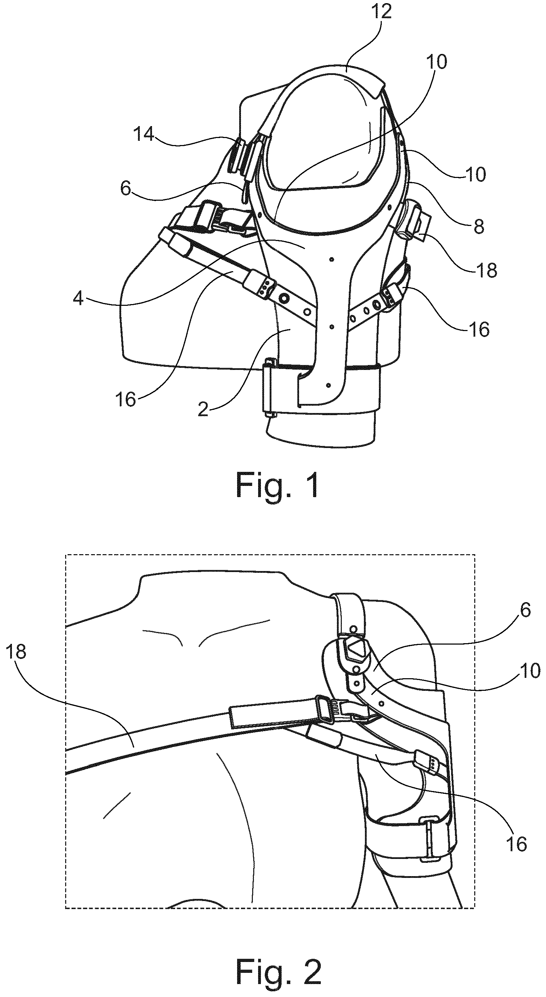

Figur 1 zeigt eine Orthese gemäß einem ersten Ausführungsbeispiel der vorliegenden Erfindung. Sie verfügt über eine formstabile Schale 2, an der ein Versteifungselement 4 angeordnet ist. Die formstabile Schale verfügt über eine ventrale Lasche 6 und eine dorsale Lasche 8. Auch Versteifungselement 4 verfügt über zwei Vorsprünge 10, die sich entlang der beiden Laschen 6, 8 erstrecken. Oberhalb der Schulter sind die beiden Laschen 6, 8 durch einen Gurt 12 miteinander verbunden. An diesem Gurt 12 befindet sich ein Betätigungselement 14, das im gezeigten Ausführungsbeispiel als Drehknopf ausgebildet ist. Es ist eingerichtet, ein Spannelement zu spannen, das in den Figuren nicht dargestellt ist. Über zwei zusätzliche Haltegurte 16, die die formstabile Schale 2 mit einem Oberkörpergurt 18 verbinden, wird ein Bewegungsspielraum des Oberarms bei angelegter Orthese weiter eingeschränkt.

Figur 2 zeigt die Orthese aus Figur 1 in einer anderen Ansicht. Man erkennt nun besser den Oberkörpergurt 18, an dem einer der beiden Haltegurte 16 befestigt ist. Zudem ist gut zu erkennen, dass die ventrale Lasche 6 sich vom Oberarm des Trägers ausgehend nach proximal erstreckt und sich entlang dieser ventralen Lasche 6 einer der beiden Vorsprünge 10 des Versteifungselementes 4 erstreckt.

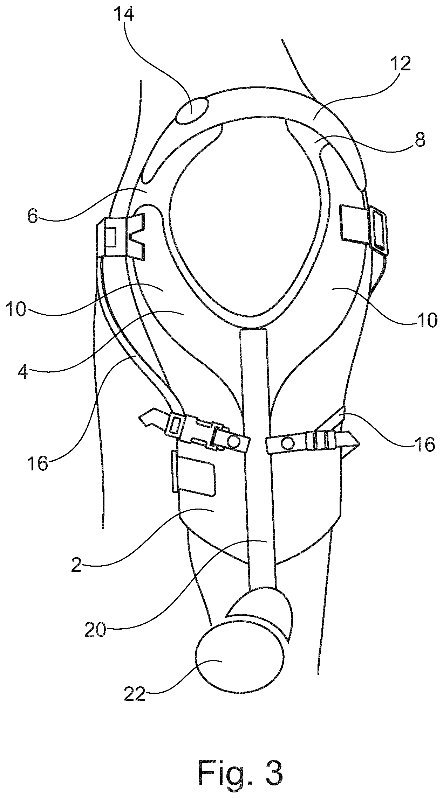

Figur 3 zeigt eine Seitenansicht einer anderen Ausgestaltung einer Orthese gemäß der vorliegenden Erfindung. An dem Versteifungselement 4, das die beiden Vorsprünge 10 aufweist, die sich entlang der ventralen Lasche 6 und der dorsalen Lasche 8 erstrecken. Die beiden Laschen 6, 8 sind wieder Teile der formstabilen Schale 2. Auch die beiden Haltegurte 16 sind vorhanden. Über der Schulter sind die beiden Laschen 6, 8 mit dem Gurt 12 verbunden, an dem sich das Betätigungselement 14 für das Spannelement befindet. Im distalen Bereich der formstabilen Schale 2 ist an dem Versteifungselement 4 ein Schienenelement 20 angeordnet, an dessen unterem Ende ein Gelenk 22 eines Schienensystems angeordnet ist.

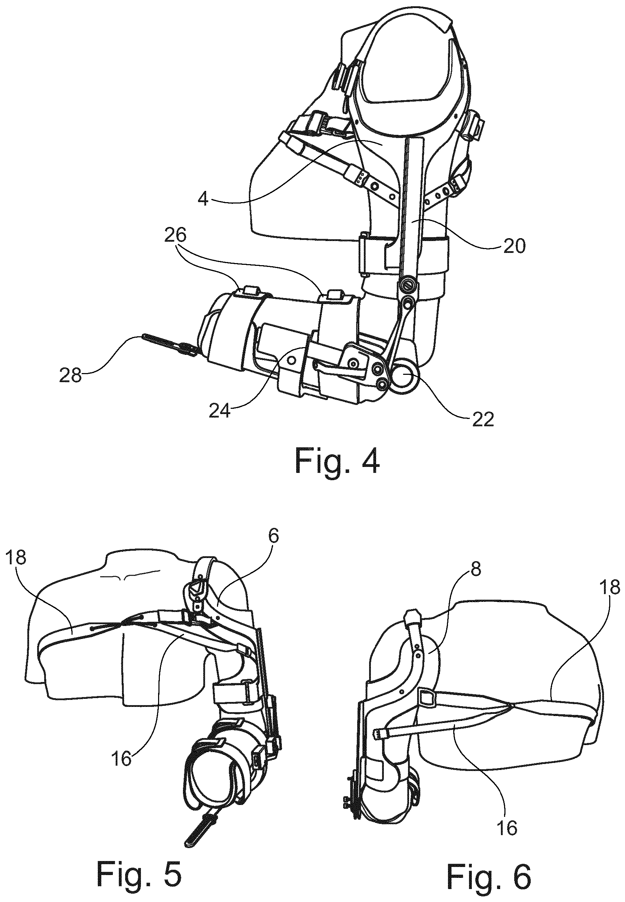

Figur 4 zeigt eine Seitenansicht einer weiteren Ausgestaltung einer Orthese gemäß der vorliegenden Erfindung. An dem Versteifungselement 4 befindet sich das Schienenelement 20 mit dem daran angeordneten Gelenk 22. Dieses Gelenk 22 verbindet im gezeigten Ausführungsbeispiel das Schienenelemen22, das im angelegten Zustand entlang des Oberarms des Trägers verläuft mit einer Unterarmschiene 24, die entlang des Unterarms verläuft. Das Gelenk 22 ist vorzugsweise derart an dem Schienenelement 20 und der Unterarmschiene 24 angeordnet, dass seine Schwenkachse parallel und vorzugsweise koaxial zu der Schwenkachse des Ellenbodengelenkes des Trägers verläuft. Über Unterarmgurte 26 lässt sich die Unterarmschiene 24 am Unterarm befestigen, so dass ein fester und sicherer Halt der Orthese auch am Unterarm gewährleistet ist. Die Orthese ist durch das Gelenk 22 und die dadurch verbundenen Schienen 20, 24 eingerichtet, eine Kraft auf den Unterarm aufzubringen, die Bewegung des Ellenbodengelenkes des Trägers zu unterstützen, zu begrenzen und/oder zu trainieren und/oder das Ellenbogengelenk zu stützen, zu entlasten und/oder zu schützen. An der Unterarmschiene 24 ist ein Befestigungselement 28 angeordnet, an dem ein Handstützelement, das nicht gezeigt ist, angeordnet werden kann. So lässt sich die gezeigte Orthese mit einer an sich bekannten Handorthese kombinieren.

Figuren 5 und 6 zeigen die Orthese aus Figur 4 in anderen Ansichten. Figur zeigt eine Frontalansicht. Dabei sind besonders der um den Oberkörper herum geführte Oberkörpergurt 18 und der die Bewegung des Oberarms einschränkende Haltegurt 16 gut zu erkennen. In der in Figur 6 dargestellten Rückansicht ist gezeigt, dass der Oberkörpergurt 18 um den Oberkörper herum geführt ist. Er verbindet vorzugsweise die ventrale Lasche 6 mit der dorsalen Lasche 8. Auch im rückwärtigen Bereich verfügt die Orthese im gezeigten Ausführungsbeispiel über einen Haltegurt 16.

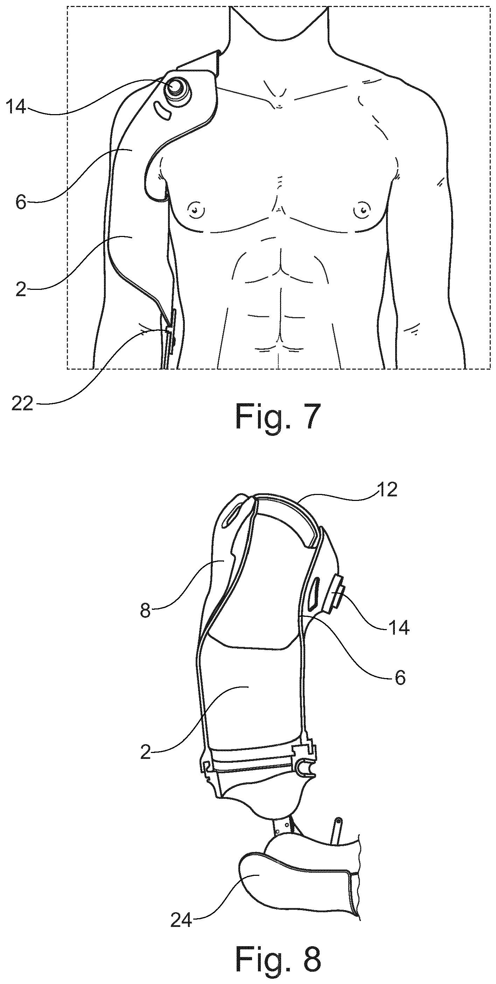

Figur 7 zeigt eine Orthese gemäß einem weiteren Ausführungsbeispiel der vorliegenden Erfindung im angelegten Zustand. Figur 8 zeigt die gleiche Orthese im nicht angelegten Zustand in einer lateralen Ansicht. Die ventrale Lasche 6 und die dorsale Lasche 8 sind jeweils als Teil der formstabilen Schale 2 ausgebildet. Die Schale 2 und die beiden Laschen 6,8 sind als Bauteile aus Kohlefaserverbundmaterial (CFK) hergestellt, was ihnen eine ausreichende mechanische Stabilität bei wenig Eigengewicht verleiht. Über die Schulter des Trägers verläuft der Gurt 12. Das Spannelement 14, durch das die wirksame Länge des Zugseils verändert werden kann. Ein solches Element wird beispielsweise von der Firma BOA angeboten. Die Schale 2 ist medial angeordnet, erstreckt sich also zwischen dem Arm und dem Oberkörper des Benutzers. Auch das Gelenk 22 ist medial am Arm positioniert.

Am distalen Ende der formstabilen Schale 2 ist über ein Gelenk 22 eine Unterarmschiene 24 angeordnet, die in der gezeigten Ausführungsform als CFK-Bauteil ausgestaltet ist. Die CFK-Bauteile sind mit einer Polsterschicht versehen, die dem Körperteil zugewandt angeordnet ist.

Man erkennt insbesondere in Figur 8, dass die Schale zweiteilig ausgebildet ist. Sie weist einen oberen oder proximalen Anteil und einen unteren oder distalen Anteil auf. Beide Anteile sind durch eine Bewegungseinrichtung miteinander verbunden, die die beiden Anteile gegeneinander verschwenkbar miteinander verbindet. Damit ist es möglich, einen Unterarm, an dem die Unterarmschiene positioniert, in einem vorbestimmten Winkelbereich zu verschwenken. Damit ist beispielsweise eine Position des Unterarms relativ zur Schulter einstellbar. Der Unterarm ist dabei sowohl durch eine externe Kraft, beispielsweise durch eine Bewegung durch den anderen Arm des Benutzers, oder durch eine Bewegung aus eigenem Antrieb bewegbar.

### Bezugszeichenliste

- 2: formstabile Schale
- 4: Versteifungselement
- 6: ventrale Lasche
- 8: dorsale Lasche
- 10: Vorsprung
- 12: Gurt
- 14: Betätigungselement
- 16: Haltegurt
- 18: Oberkörpergurt
- 20: Schienenelement
- 22: Gelenk
- 24: Unterarmschiene
- 26: Unterarmgurt
- 28: Befestigungselement

## Patentansprüche

1. Verfahren zum Befestigen einer Schulterorthese an einem Oberarm, wobei die Schulterorthese eine formstabile Schale (2) mit einer ventralen Lasche (6) und einer dorsalen Lasche (8) aufweist, wobei die ventrale Lasche (6) und die dorsale Lasche (8) mittels eines Spannelementes verbunden oder verbindbar sind, wobei das Verfahren folgende Schritte aufweist:
- Anlegen der formstabilen Schale (2) an einen Oberarm,
- Spannen des Spannelementes, sodass eine Zugkraft auf die ventrale Lasche (6) und die dorsale Lasche (8) ausgeübt wird, die Richtung proximal wirkt.

2. Verfahren nach Anspruch 1, **dadurch gekennzeichnet, dass** die ventrale Lasche (6) und die dorsale Lasche (8) vor dem Spannen des Spannelementes miteinander verbunden, vorzugsweise aneinander befestigt, werden.

3. Verfahren nach Anspruch 2, **dadurch gekennzeichnet, dass** die ventrale Lasche (6) und die dorsale Lasche (8) oberhalb der Schulter miteinander verbunden, vorzugsweise aneinander befestigt werden.

4. Schulterorthese zum Durchführen eines Verfahrens nach einem der vorstehenden Ansprüche, wobei die Schulterorthese eine formstabile Schale (2) mit einer ventralen Lasche (6) und einer dorsalen Lasche (8) aufweist, wobei die Schulterorthese ein Spannelement aufweist, **dadurch gekennzeichnet, dass** durch das Spannelement eine Zugkraft auf die ventrale Lasche (6) und die dorsale Lasche (8) aufbringbar ist, wobei die Zugkraft vergrößerbar ist, indem das Spannelement gespannt wird.

5. Schulterorthese nach Anspruch 4, **dadurch gekennzeichnet, dass** das Spannelement einen Gurt (12) aufweist, der mit einem ersten Ende an einer der Laschen (6, 8) befestigt ist, mit einem zweiten Ende an der Lasche (6, 8) befestigbar ist und durch eine Umlenkschlaufe an der anderen Lasche (8, 6) geführt wird.

6. Schulterorthese nach Anspruch 4 oder 5, **dadurch gekennzeichnet, dass** das Spannelement ein Zugseil aufweist, das mit der ventralen Lasche (6) und der dorsalen Lasche (8) verbunden ist und dessen wirksame Länge veränderbar ist.

7. Schulterorthese nach Anspruch 6, **dadurch gekennzeichnet, dass** das Zugseil durch wenigstens eine Schlaufe an der ventralen Lasche (6) und/oder wenigstens eine Schlaufe an der dorsalen Lasche (8) geführt ist.

8. Schulterorthese nach einem der Ansprüche 4 bis 7, **dadurch gekennzeichnet, dass** an der formstabilen Schale (2) ein Versteifungselement (4) angeordnet ist.

9. Schulterorthese nach Anspruch 8, **dadurch gekennzeichnet, dass** das Versteifungselement (4) aus einem Metall, beispielsweise Aluminium oder Stahl, oder aus einem Kunststoff, einem faserverstärkten Kunstsoff, beispielsweise GFK oder CFK, hergestellt ist.

10. Schulterorthese nach Anspruch 8 oder 9, **dadurch gekennzeichnet, dass** das Versteifungselement (4) wenigstens einen, vorzugsweise zwei Vorsprünge (10) aufweist, die an der dorsalen und/oder der ventralen Lasche (8, 6) angeordnet sind.

11. Schulterorthese nach einem der Ansprüche 4 bis 10, **dadurch gekennzeichnet, dass** an der formstabilen Schale (2) und/oder an dem Versteifungselement (4) ein Element (20) eines orthopädietechnischen Schienensystems befestigt oder befestigbar ist.

12. Schulterorthese nach Anspruch 11, **dadurch gekennzeichnet, dass** das Schienensystem Teil der Schulterorthese ist und wenigstens ein Körperanlageelement, vorzugsweise ein Unterarmelement zum Anlegen an einen Unterarm und/oder ein Handelement zum Anlegen an eine Hand aufweist.

13. Schulterorthese nach Anspruch 11 oder 12, **dadurch gekennzeichnet, dass** das Schienensystem wenigstens zwei Elemente aufweist, die mittels eines Gelenkes (22) aneinander angeordnet sind.

14. Schulterorthese nach Anspruch 11, 12 oder 13, **dadurch gekennzeichnet, dass** an dem Schienensystem wenigstens ein Aktuator angeordnet ist, der eingerichtet ist, ein Element des Schienensystems relativ zu einem anderen Element des Schienensystems zu bewegen.

15. Schulterorthese nach einem der Ansprüche 4 bis 14, **dadurch gekennzeichnet, dass** die geometrische Form der formstabilen Schale (2) an den Oberarm angepasst ist.

16. Schulterorthese nach einem der Ansprüche 4 bis 15, **dadurch gekennzeichnet, dass** an einer dem Oberarm zugewandten Seite der formstabilen Schale (2) ein flexibles, vorzugsweise elastisches, Polsterelement angeordnet ist, das vorzugsweise ein textiles Polsterelement ist.

## Claims

1. A method for fastening an shoulder orthesis to an upper arm, the shoulder orthosis comprising a dimensionally stable shell (2) with a ventral tab (6) and a dorsal tab (8), wherein the ventral tab (6) and the dorsal tab (8) are or can be connected by means of a tensioning element, the method comprising the following steps:
- applying the dimensionally stable shell (2) to an upper arm,
- tensioning the tensioning element so that a tensile force is exerted on the ventral tab (6) and the dorsal tab (8), said force acting in the proximal direction.

2. The method according to claim 1, **characterized in that** the ventral tab (6) and the dorsal tab (8) are connected to each other, preferably fastened to each other, before the tensioning element is tensioned.

3. The method according to claim 2, **characterized in that** the ventral tab (6) and the dorsal tab (8) are connected to each other, preferably fastened to each other, above the shoulder.

4. A shoulder orthosis for carrying out a method according to one of the preceding claims, the shoulder orthosis comprising a dimensionally stable shell (2) with a ventral tab (6) and a dorsal tab (8), wherein the shoulder orthosis comprises a tensioning element **characterized in that** by the tensioning element a tensile force can be applied to the ventral tab (6) and the dorsal tab (8), wherein the tensile force can be increased by tensioning the tensioning element.

5. The shoulder orthosis according to claim 4, **characterized in that** the tensioning element comprises a strap (12) that is fastened at one end to one of the tabs (6, 8), that can be fastened at the second end to the tab (6, 8) and that is guided through a guide loop on the other tab (8, 6).

6. The shoulder orthosis according to claim 4 or 5, **characterized in that** the tensioning element has a tensioning cord, which is connected to the ventral tab (6) and the dorsal tab (8) and the effective length of which can be altered.

7. The shoulder orthosis according to claim 6, **characterized in that** the tensioning cord is guided through at least one loop on the ventral tab (6) and/or at least one loop on the dorsal tab (8).

8. The shoulder orthosis according to one of the claims 4 to 7, **characterized in that** a reinforcement element (4) is arranged on the dimensionally stable shell (2).

9. The shoulder orthosis according to claim 8, **characterized in that** the reinforcement element (4) is made of a metal, such as aluminium or steel, or a plastic or a fiber-reinforced plastic, such as GRP or CFRP.

10. The shoulder orthosis according to claim 8 or 9, **characterized in that** the reinforcement element (4) comprises at least one, preferably two, projections (10), which are arranged on the dorsal and/or on the ventral tab (8, 6).

11. The shoulder orthosis according to one of the claims 4 to 10, **characterized in that** an element (20) of an orthopedic splint system is or can be fastened to the dimensionally stable shell (2) and/or the reinforcement element (4).

12. The shoulder orthosis according to claim 11, **characterized in that** the splint system is part of the shoulder orthosis and comprises at least one body contact element, preferably a lower arm element to be applied to a lower arm and/or a hand element to be applied to a hand.

13. The shoulder orthosis according to claim 11 or 12, **characterized in that** the splint system comprises at least two elements that are arranged together by means of a joint (22).

14. The shoulder orthosis according to claim 11, 12 or 13, **characterized in that** at least one actuator is arranged on the splint system, said actuator being configured to move an element of the splint system relative to another element of the splint system.

15. The shoulder orthosis according to one of the claims 4 to 14, **characterized in that** the geometric form of the dimensionally stable shell (2) is adapted to the upper arm.

16. The shoulder orthosis according to one of the claims 4 to 15, **characterized in that** a flexible, preferably elastic, padding element is arranged on a side of the dimensionally stable shell (2) facing the upper arm, said padding element preferably being a textile padding element.

## Revendications

1. Procédé de fixation d'une orthèse d'épaule à un bras, l'orthèse d'épaule comportant une coque solide en forme (2) munie d'une patte ventrale (6) et d'une patte dorsale (8), la patte ventrale (6) et la patte dorsale (8) étant reliées ou pouvant être reliées au moyen d'un élément tendeur, le procédé comprenant les étapes suivantes consistant à :
- appliquer la coque solide en forme (2) sur un bras,
- tendre l'élément tendeur de manière à exercer une force de traction sur la patte ventrale (6) et sur la patte dorsale (8), laquelle agit dans le sens proximal.

2. Procédé selon la revendication 1,
**caractérisé en ce que** la patte ventrale (6) et la patte dorsale (8) sont reliées l'une à l'autre, de préférence fixées l'une à l'autre, avant que l'élément tendeur ne soit tendu.

3. Procédé selon la revendication 2,
**caractérisé en ce que** la patte ventrale (6) et la patte dorsale (8) sont reliées l'une à l'autre, de préférence fixées l'une à l'autre, au-dessus de l'épaule.

4. Orthèse d'épaule pour mettre en œuvre un procédé selon l'une des revendications précédentes, l'orthèse d'épaule comportant une coque solide en forme (2) munie d'une patte ventrale (6) et d'une patte dorsale (8), l'orthèse d'épaule comprenant un élément tendeur,
**caractérisée en ce que** l'élément tendeur permet d'exercer une force de traction sur la patte ventrale (6) et sur la patte dorsale (8), la force de traction pouvant être augmentée en tendant l'élément tendeur.

5. Orthèse d'épaule selon la revendication 4,
**caractérisée en ce que** l'élément tendeur comprend une sangle (12) dont une première extrémité est fixée à l'une des pattes (6, 8) et dont une deuxième extrémité peut être fixée à la patte (6, 8) en la faisant passer à travers une boucle de renvoi sur l'autre patte (8, 6).

6. Orthèse d'épaule selon la revendication 4 ou 5,
**caractérisée en ce que** l'élément tendeur comporte un câble de traction qui est relié à la patte ventrale (6) et à la patte dorsale (8) et dont la longueur effective est variable.

7. Orthèse d'épaule selon la revendication 6,
**caractérisée en ce que** le câble de traction passe à travers au moins une boucle sur la patte ventrale (6) et/ou à travers au moins une boucle sur la patte dorsale (8).

8. Orthèse d'épaule selon l'une des revendications 4 à 7,
**caractérisée en ce qu'**un élément de renfort (4) est disposé sur la coque solide en forme (2).

9. Orthèse d'épaule selon la revendication 8,
**caractérisée en ce que** l'élément de renfort (4) est fabriqué en métal, par exemple en aluminium ou en acier, ou en matière plastique, en matière plastique renforcée de fibres, par exemple en PRV ou en PRFC.

10. Orthèse d'épaule selon la revendication 8 ou 9,
**caractérisée en ce que** l'élément de renfort (4) comporte au moins une, de préférence deux saillies (10) qui sont disposées sur la patte dorsale et/ou ventrale (8, 6).

11. Orthèse d'épaule selon l'une des revendications 4 à 10,
**caractérisée en ce qu'**un élément (20) d'un système d'attelle orthopédique est fixé ou peut être fixé à la coque solide en forme (2) et/ou à l'élément de renfort (4).

12. Orthèse d'épaule selon la revendication 11,
**caractérisée en ce que** le système d'attelle fait partie de l'orthèse d'épaule et comporte au moins un élément d'application sur le corps, de préférence un élément d'avant-bras, destiné à être appliqué sur un avant-bras, et/ou un élément de main, destiné à être appliqué sur une main.

13. Orthèse d'épaule selon la revendication 11 ou 12,
**caractérisée en ce que** le système d'attelle comprend au moins deux éléments qui sont reliés l'un à l'autre au moyen d'une articulation (22).

14. Orthèse d'épaule selon la revendication 11, 12 ou 13,
**caractérisée en ce qu'**au moins un actionneur est disposé sur le système d'attelle, lequel est conçu pour déplacer un élément du système d'attelle par rapport à un autre élément du système d'attelle.

15. Orthèse d'épaule selon l'une des revendications 4 à 14,
**caractérisée en ce que** la forme géométrique de la coque solide en forme (2) est adaptée au bras.

16. Orthèse d'épaule selon l'une des revendications 4 à 15,
**caractérisée en ce qu'**un élément de rembourrage flexible, de préférence élastique, qui est de préférence un élément de rembourrage textile, est disposé sur un côté de la coque solide en forme (2) tourné vers le bras.
